# EUROPEAN PATENT APPLICATION

(11) **EP 2 272 830 A1**
(43) Date of publication of application: **12.01.2011**
(21) Application number: 09382097.5
(22) Date of filing: 18.06.2009
(51) Int. Cl.: C07D 213/42

(54) **Preparation process of an antivirally heterocyclic azahexane derivative**

(71) Applicant: Esteve Química, S.A., 08024 Barcelona (ES)
(72) Inventor: Bartra Sanmartí, Martí, 08024, Barcelona (ES); Berenguer Maimó, Ramón, 08024, Barcelona (ES); Ortiz Gil, Jordi, 08024, Barcelona (ES); Isern Sánchez, Olga, 08024, Barcelona (ES)
(74) Representative: ZBM Patents

(57) **Abstract**

A process for the preparation of atazanavir or its pharmaceutically salts, or its solvates, which comprises condensing N-(methoxycarbonyl)-L-tert-leucine and 1-[4-(piridyn-2-yl)phenyl-4(S)-hydroxy]-5-(S)-2,5-diamino-6-phenyl-2- azahexane or a salt thereof using N,N-diisopropylcarbodiimide or N,N-dicyclohexylcarbodiimide and a tertiary organic amine, being the condensation carried out in the absence of 1-hydroxy-benzotriazole.

## Description

The present invention relates to a process for the preparation of an antivirally heterocyclic azahexane derivative known as atazanavir.

### BACKGROUND ART

Atazanavir is the International Non-proprietary Name (INN) of *N*-[(1*S*)-1-{[(2*S*,3*S*)-3-hydroxy-4-[(2*S*)-2-[(methoxycarbonyl)amino]-3,3-dimethyl-*N*'-{[4-(pyridin-2-yl)phenyl]methyl}butanehydrazido]-1-phenylbutan-2-yl]carbamoyl}-2,2-dimethylpropyl]carbamate, and CAS No. 198904-31-3. Atazanavir sulfate is marketed under the trade name Reyataz by Bristol-Myers Squibb. It is an antiretroviral drug of the protease inhibitor (PI) class. Like other antiretrovirals, it is used to treat infection of human immunodeficiency virus (HIV). Atazanavir sulfate is distinguished from other Pls in that it can be given once-daily (rather than requiring multiple doses per day) and has lesser effects on the patient's lipid profile. Like other protease inhibitors, it is used only in combination with other HIV.

The structure of atazanavir sulfate corresponds to formula (I):

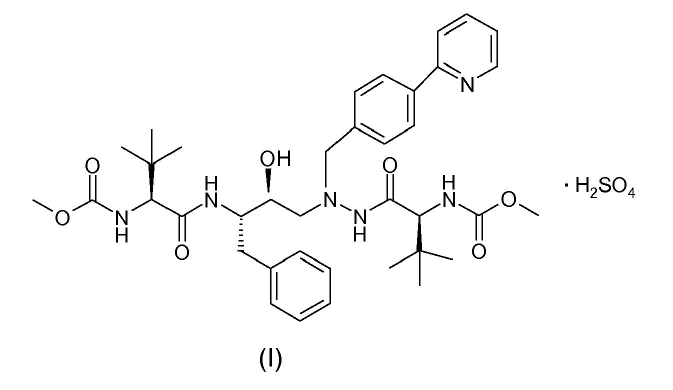

Different synthetic strategies for the preparation of atazanavir and its salts are known.

EP900210-A describes several processes for the preparation of antivirally active heterocyclic azahexane derivatives including atazanavir. One of the processes is based on a condensation of an amino compound with an acid compound or with a reactive acid derivative thereof, which may be effected in the presence of common condensing agents, a base and an appropriate solvent. Among the condensing agents are N,N'-disubstituted carbodiimides, for example N,N'-dicyclohexylcarbodiimide or especially N-(3-dimethylaminopropyl)-N'-ethylcarbodiimide. EP900210 does not describe any specific example for the preparation of atazanavir through the activation of the corresponding acid with an N,N'-disubstituted carbodiimide. Example 46 describes the coupling of N-(methoxycarbonyl)-L-tert-leucine and 1-[4-(piridyn-2-yl)phenyl-4(S)-hydroxy]-5-(S)-2,5-diamino-6-phenyl-2-azahexane trihydrochloride using an alternative reaction system which is O-(2-oxo-1(2H)pyridyl)-N,N,N',N'-tetramethyluronium tetrafluoroborate / dichloromethane / diisoproprilethylamine (TPTU/CH₂Cl₂/DIPEA). However, the use of these reaction conditions has several drawbacks. On the one hand, TPTU is not available at industrial scale and, on the other hand, the two possible isolation known processes of this preparation process are complicated and not-easily industrializable.

Carbodiimides are well known as activating agents. The reaction of a carbodiimide with a carboxylic acid yields to a highly reactive O-acyl-urea. To enhance the electrophilicity of carboxylate group, the negatively charged oxygen must first be activated into a better leaving group. Carbodiimides are used for this purpose. The negatively charged oxygen will act as a nucleophile, attacking the central carbon in the carbodiimide. The carbodiimide is temporarily attached to the former carboxylate group, which is now an ester group, making nucleophilic attack by an amino group (on the attaching amino acid) to the former C-terminus (carbonyl group) more efficient. The problem with carbodiimides is that they are too reactive and that they can therefore cause racemization of the amino acid.

To solve the problem of racemization, triazolols were introduced. The most important ones are 1-hydroxy-benzotriazole (HOBt) and 1-hydroxy-7-azabenzotriazole (HOAt). These substances avoid the racemization, mostly because the reaction is highly accelerated by their addition to the reaction mixture. They can react with the O-acylurea to form an active ester which is more stable and less in danger of racemization. Thus, these substances have not only the effect of acting as racemization suppressors but also of improving the yield in this type of coupling.

Zhongmin Xu et al. describes the coupling between N-(methoxycarbonyl)-L-tert-leucine and 1-[4-(piridyn-2-yl)phenyl-4(S)-hidroxy]-5-(S)-2,5-diamino-6-phenyl-2-azahexane trihydrochloride using a carbodiimide soluble in water, 1-(3-dimethylaminopropyl)-3-ethylcarbodiimide hydrochloride (WSC), diisopropylethylamine, and HOBt (cf. Organic Process Research & Development 2002, vol. 6, pp. 323-328). US20050256314 also describes the coupling between N-(methoxycarbonyl)-L-tert-leucine and 1-[4-(piridyn-2-yl)phenyl-4(S)-hidroxy]-5-(S)-2,5-diamino-6-phenyl-2-azahexane trihydrochloride using HOBt and WSC in dichloromethane as solvent to yield atazanavir.

EP 1930324-A describes the use of DCC and HOBt for the coupling of one of the side chains of the atazanavir. The process described in this document is based on the sequential introduction of the side chains and include steps of introduction and elimination of protecting groups.

Recently, HOBt has been removed from many chemical vendor catalogues; although almost always found as a hydrate, HOBt may be explosive when allowed to fully dehydrate and shipment by air or sea is heavily restricted.

Newer developments omit the carbodiimides totally to avoid the use of HOBt. The active ester is introduced as a uronium or phosphonium salt of a non-nucleophilic anion (tetrafluoroborate or hexafluorophosphate) such as O-(benzotriazol-1-yl)-N,N,N',N'-tetramethyluronium hexafluorophosphate (HBTU), O-(7-azabenzotriazol-1-yl)-N,N,N',N'-tetramethyluronium hexafluorophosphate (HATU), O-(benzotriazol-1-yl)-N,N,N',N'-tetramethyluronium tetrafluoroborate (TBTU), or (benzotriazol-1-yloxy)tripyrrolidinophosphonium hexafluorophosphate (PyBOP). These reagents have the drawback of their high price. In terms of atom economy they are products not attractive for activating carboxylic acids.

From the previous documents it is derived that the presence of HOBt is essential for the activation of the corresponding acid as an N,N'-disubstituted amidino esters in order to obtain similar results to those obtained with TPTU regarding to yield and purity.

Thus, from what is known in the art it is derived that there is still the need of providing efficient and environmentally suitable processes for the preparation of atazanavir. Therefore, the provision of a new easily industrializable process for the preparation of atazanavir and its salts, which proceeds without racemization and with high yield and purity, would be of great interest in industry.

### SUMMARY OF THE INVENTION

Inventors have found that the coupling between N-(methoxycarbonyl)-L-tert-leucine and 1-[4-(piridyn-2-yl)phenyl-4(S)-hydroxy]-5-(S)-2,5-diamino-6-phenyl-2-azahexane or a salt thereof can be carried out efficiently using N,N-diisopropylcarbodiimide (DIC) or N,N-dicyclohexylcarbodiimide (DCC) and a tertiary organic amine in the absence of HOBt without causing racemization.

Both carbodiimides, DIC and DCC, are structurally related. They have also in common that are not functionalized. The formula of each one is shown below:

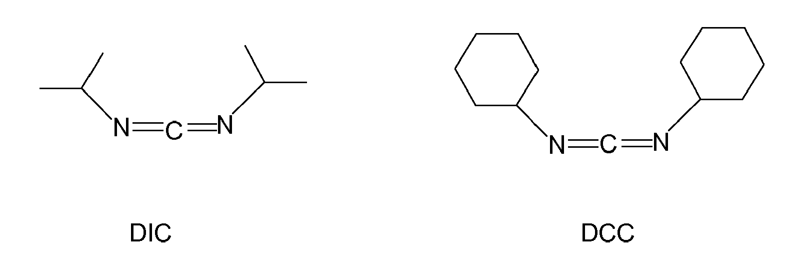

The absence of HOBt in the coupling makes easy and safe the isolation of the product since it avoids working with an explosive and allergenic reactive which is difficult to eliminate. Thus, the process of the present invention is advantageous since it means the introduction of a cleaner and safe process in industry by avoiding the use of environmentally damaging compounds, which in today's society has become a major concern. It is also advantageous in terms of atom economy. In addition, the atazanavir obtained has a high purity and is obtained with high yield. As it is illustrated in the Examples, by using other carbodiimides, for instance 1-(3-dimethylaminopropyl)-3-ethylcarbodiimide hydrochloride (WSC), a high conversion is obtained in the presence of HOBt, but no conversion is produced in the absence of HOBt. The unexpected results of the present invention are assumed to be consequence of the selection of the specific combination of the carbodiimide and the tertiary organic amine.

Thus, according to an aspect of the present invention it is provided a process for the preparation of atazanavir of formula (I), or its pharmaceutically salts, or its solvates, which comprises condensing a compound of formula (II) or a salt thereof, with a compound of formula (III) using a carbodiimide selected from the group consisting of N,N-diisopropylcarbodiimide and N,N-dicyclohexylcarbodiimide, and a tertiary organic amine, in the absence of 1-hydroxy-benzotriazole.

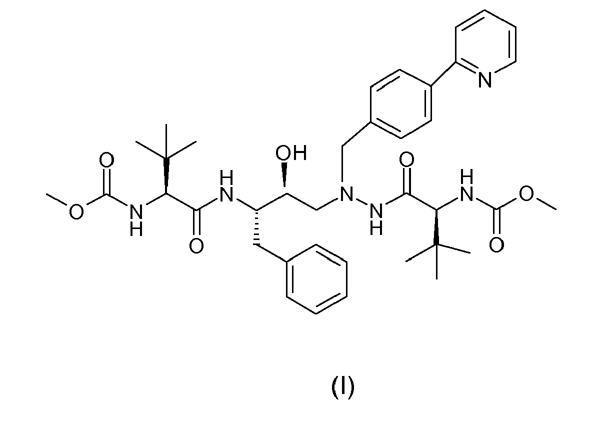

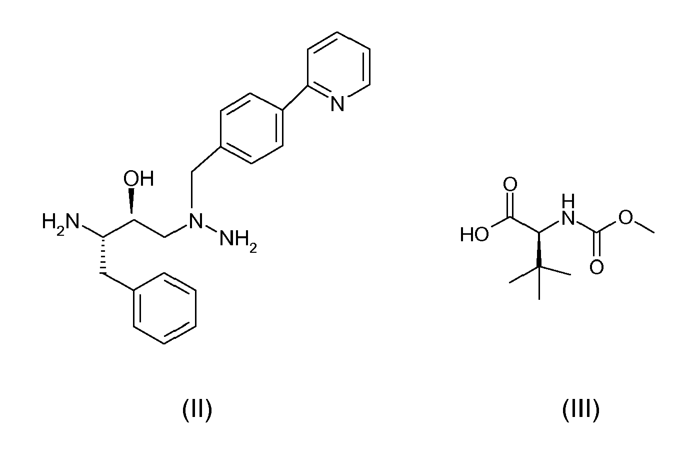

### DETAILED DESCRIPTION OF THE INVENTION

As mentioned above, the coupling between a compound of formula (II) or a salt thereof with a compound of formula (III) to yield atazanavir can be carried out using a carbodiimide selected from DIC and DCC and a tertiary organic amine, in the absence of HOBt.

In a preferred embodiment, the carbodiimide is DIC. DIC is a liquid and is easy to handle. In addition the N,N'-diisopropylurea formed as a subproduct is partially insoluble in organic solvents and most of it can be easily removed, for instance, by filtration. Likewise, as it is illustrated in the Examples, the best results regarding to yield and purity are obtained when the coupling is carried out with DIC and an organic tertiary amine, in the absence of HOBt.

In a more preferred embodiment, the tertiary organic amine is selected from N-methylmorpholine (NMM), triethylamine (TEA), and N,N-diisopropylethylamine (DIPEA). In a still more preferred embodiment, the tertiary organic amine is N-methylmorpholine or triethylamine. A minimum amount of 2 equivalents of tertiary organic amine per equivalent of compound of formula (II) is needed. The minimum amount can be higher when compound (II) is in form of a salt. For instance, when the trihydrochloride salt of compound (II) is used as starting material, then a minimum amount of 5 equivalents of tertiary organic amine per equivalent of compound (II) is needed since three equivalents are used to convert the trihydrochloride salt of compound (II) into the compound (II) as free base. In general, the amount of the tertiary organic amine is comprised between 2-20 equivalents per equivalent of compound of formula (II). Preferably, the amount of the tertiary organic amine is comprised between 2-8 equivalents per equivalent of compound of formula (II). When the trihydrochloride salt of compound (II) is used as starting material, preferably, the amount of the tertiary organic amine is comprised between 5-11 equivalents per equivalent of trihydrochloride salt of compound (II). More preferably, the amount of the tertiary organic amine is comprised between 6-8 equivalents per equivalent of the trihydrochloride of compound of formula (II). In a particular embodiment, the amount of tertiary organic amine is 6.6 equivalents per equivalent of the trihydrochloride of compound of formula (II).

Generally, an amount of 2 equivalents or higher of compound of formula (III) per equivalent of compound of formula (II) is used. In general, the amount of compound (III) is comprised between 2-20 equivalents per equivalent of compound of formula (II), although higher amounts can also be used. It has been found that specially good results are obtained by carrying out the coupling with at least 2.5 equivalents, and even better, with at least 3 equivalents of compound (III) per equivalent of compound (II). In a preferred embodiment, the coupling is carried out with an amount of compound (III) comprised between 3-5 equivalents per equivalent of compound (II). In a more preferred embodiment, the reaction is carried out with 3.5 equivalent of compound (III) per equivalent of compound (II).

Generally, an amount of 2 equivalents or higher of carbodiimide per equivalent of compound of formula (II) is used. In a preferred embodiment, the amount of carbodiimide is comprised between 2-20 equivalents per equivalent of compound of formula (II). In a more preferred embodiment, the amount of carbodiimide is comprised between 2-5 equivalents per equivalent of compound (II). In a still more preferred embodiment, the amount of carbodiimide is comprised between 2.5-5 equivalents per equivalent of compound (II). In a particular embodiment, the amount of carbodiimide is 2.5 equivalents per equivalent of compound (II). In another particular embodiment the amount of carbodiimide is 3.5 equivalents per equivalent of compound (II).

The preparation process can be carried out in the presence of an appropriate solvent. Examples of appropriate solvents include (C₁-C₆)-chlorine containing solvents such as dichloromethane or chloroform, (C₆-C₈)-aromatic hydrocarbons such as toluene or xylene, and (C₁-C₆)-alkyl (C₂-C₆)-alkanoates such as ethyl acetate.

The coupling can be carried out at a temperature comprised between -20 °C and 30 °C approximately. Generally, the coupling is carried out at room temperature.

Once the reaction is completed, the isolation of the atazanavir can be carried out by a process comprising the filtration of the subproducts formed, followed by one or more washings with an aqueous medium in order to separate the salts, removal of the reaction solvent and finally addition of an appropriate solvent from which atazanavir can be crystallized. An example of an appropriate solvent is tert-butylmethylether (TBME). The atazanavir obtained can be isolated by filtration. The most adequate conditions for carrying out said isolation process vary depending on the parameters considered by an expert in the art, such as the concentration, the temperature, the solvent used during the reaction or the isolation of the product, and the like. These can be readily determined by said skilled person in the art with the help of the teachings of the examples given in this description.

The atazanavir can be isolated as a pharmaceutically acceptable salt. A salt can be obtained from the atazanavir base by treatment with the corresponding acid. Alternatively, a salt of the atazanavir obtainable according to the process of the invention is converted into another salt. Preferably, the salt is the sulfate salt which is obtained by reaction of atazanavir with sulfuric acid in a molar ratio 1:1.

The atazanavir, including their salts, may also be obtained in the form of hydrates, or their crystals may include for example the solvent used for crystallization.

Compound of formula (III) is commercial. Compound of formula (II) can be obtained from 1-[4-(pyridin-2-yl)-phenyl]-4(S)-hydroxy-5(S)-2,5-bis [(tert-butoxycarbonyl)amino]6-phenyl-2-azahexane by treatment with an acid in an appropriate solvent. Example of appropriate reaction conditions are mixtures of aqueous hydrochloric acid and tetrahydrofuran, N,N-dimethylformamide or dichloromethane, mixture of hydrogen chloride in dioxane and N,N-dimethylformamide or a solution of hydrogen chloride in isopropanol.

Throughout the description and claims the word "comprise" and variations of the word, are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1: Preparation of atazanavir using 1 equivalent of compound (II), 3.5 equivalents of compound (III), 3.5 equivalents of DIC, and 6.6 equivalents of NMM

97.5 g of 1-[4-(piridyn-2-yl)phenyl-4(S)-hydroxy]-5-(S)-2,5-diamino-6-phenyl-2-azahexane trihydrochloride (trihydrochloride of compound (II) with a 10%wt content of isopropanol, 185.97mmol) were suspended into 683 ml of dichloromethane under nitrogen atmosphere at -10 °C. 135 mL (1227.40 mmol) of N-methylmorpholine were added maintaining the temperature at -10 °C.

Separately, 100.8 mL (650.90 mmol) of N,N-diisopropylcarbodiimide were added to a suspension of 123.2 g (650.90 mmol) of N-(methoxycarbonyl)-L-tert-leucine (compound (III)) into 975 ml of dichloromethane.

Then, the first suspension was quickly transferred over the second one. The resulting mixture was warmed up to room temperature and was maintained at such temperature until the reaction was completed (93% atazanavir by HPLC, monoimpurity content: 1.2%).

The reaction mixture was filtered off and was washed with 800 mL of water. Then, the organic phase was concentrated up to half volume and 500 mL of tert-butylmethylether were added. The mixture was concentrated again up to half volume. This operation was repeated three times up to a dichloromethane content equal or less to 20%. The precipitated product was recovered by filtration. 125.4 g of atazanavir were obtained (Yield =96%). Purity by High Performance Liquid Chromatography (HPLC) = 98.2%, with 4% of N,N-diisopropylurea (DIU) and free of the other probable diastereomers. Molar yield =92%. Recrystallization in ethanol/water 45:55 yielded 105.34 g of atazanavir (149.5 mmol). Recrystallization yield: 84%. Purity HPLC = 99.4%, free of DIU, and free of the other probable diastereomers.

The formula of the three probable diastereomers and the HPLC conditions to detect their presence are included below: 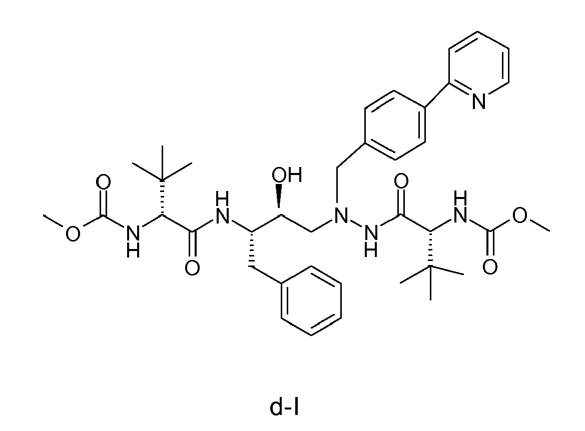 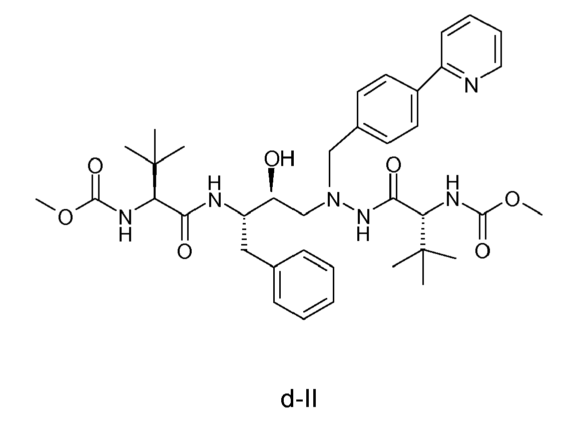 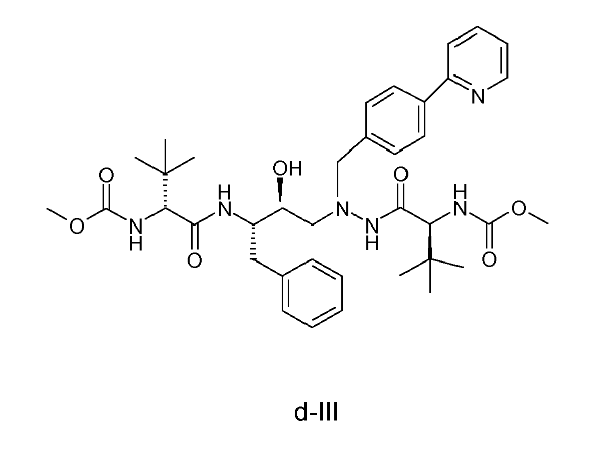
HPLC conditions:
Liquid chromatograph with UV detector equipped with automatic injector, and
integration system
Column: ZORBAX Eclipse XDB-C18 150x4.6 mm, 5µm.
Mobile phase: A (0.05% formic acid in water) and B (ACN)

**Gradient elution:**

| t (min) | % B | % A |
|---|---|---|
| 0 | 20 | 80 |
| 20 | 100 | 0 |

Detection: 254 nm
Flow: 1 mL/min
Column temperature: 25 °C
Injection: 2 µL
Time injection and chromatogram: 20 min

**Relative retention time of the diastereomers (RRT):**

| | |
|---|---|
| RRT 0.98 | d-II or d-III |
| RRT 1.00 | atazanavir |
| RRT 1.03 | d-II or d-III |
| RRT 1.08 | dI |

### Example 2: Preparation of atazanavir sulfate

A compound obtained as in Example 1 was transformed into the sulfate salt by addition of sulfuric acid following the process described in WO 9936404, in order to confirm that no racemization has been occurred following the process of the present invention. The confirmation was done by comparing the rotatory power of the atazanavir in form of sulfate salt obtained following the process of the present invention with the value known in the state of the art for atazanavir sulfate.

172.8 g (245.16 mmol) of atazanavir and some crystals of atazanavir sulfate were suspended in 1728 ml acetone at 50 °C. 49 mL of sulfuric acid 5M (245.16 mmols) were quickly added and after a few minutes precipitation of a product was observed. It was maintained first at 50 °C during 1 hour, then at 25 °C during 30 min and, finally, at 0 °C during an additional 30 min. The solid was filtered off, washed with 170 mL of acetone and 170 mL of heptane, and dried under vacuum. 186.7 g (232.52 mmol) of atazanavir sulfate were obtained (yield: 95%, purity HPLC: 99.85%). [α]_{D} -45.3 (MeOH/H₂O 1:1, 1 mg/mL). The rotatory power of atazanavir sulfate described in the literature is [α]_{D} -46.1 (cf. Z. Xu et al., Organic Process Research & Development, 2002, vol. 6, pp. 323-328) which confirms that atazanavir base or its salts can be obtained by the process of the present invention efficiently without causing racemization.

### Example 3: Preparation of atazanavir using 1 equivalent of compound (II), 3.5 equivalents of compound (111), 2.5 equivalents of DIC, and 6.6 equivalents of NMM

Following the method described in Example 1 and starting from 185.8 g of 1-[4-(piridyn-2-yl)phenyl-4(S)-hydroxy]-5-(S)-2,5-diamino-6-phenyl-2-azahexane trihydrochloride (trihydrochloride of compound (II) with a 12%wt content of isopropanol, 346.52 mmol) but with 2.5 equivalents of DIC instead of 3.5 equivalents, 93% atazanavir was obtained by HPLC (monoimpurity 0.8%). Molar yield of 93%. Purity HPLC = 98.0%.

### Example 4: Preparation of atazanavir

Atazanavir was prepared following the method described in Example 1, using 5.909 g de 1-[4-(piridyn-2-yl)phenyl-4(S)-hydroxy]-5-(S)-2,5-diamino-6-phenyl-2-azahexane trihydrochloride (trihydrochloride of compound (II), 12.52 mmol) in 40 mL of dichloromethane (CH₂Cl₂), 9.11 mL of NMM (82.65 mmol), 8.293 g of N-(methoxycarbonyl)-L-tert-leucine (III) (43.83 mmol) in 60 mL of CH₂Cl₂, 4.85 mL of DIC (31.31 mmol), 60 mL of water, and 3 X 50 mL of TBME. 7.430 g (10.54 mmol) of atazanavir were obtained. Yield:84%. Purity: 97.9%, 7% of DIU (Molar yield: 78%).

### Examples 5-13: Preparation of atazanavir in a React-Array multireactor

### A) General method using 3.5 equivalents of N-(methoxvcarbonyl)-L-tert-leucine (III)

N-(methoxycarbonyl)-L-tert-leucine (III) (1.30 mmol) was suspended into 2 ml of the corresponding solvent under nitrogen atmosphere at -10 °C and then the corresponding carbodiimide was added (0.93 mmol).

Separately, a suspension of 0.2 g of 1-[4-(piridyn-2-yl)phenyl-4(S)-hydroxy]-5-(S)-2,5-diamino-6-phenyl-2-azahexane trihydrochloride (II) (trihydrochloride of compound (II) with a 12%wt content of isopropanol, 0.37 mmol) and the corresponding amine (2.46 mmol) in an appropriate solvent (1.4 ml) under nitrogen atmosphere and at -10 °C was prepared.

Then, the first suspension was quickly transferred over the second one. The resulting mixture was warmed up to room temperature and was maintained at such temperature until the reaction was completed. The reaction control was carried out by HPLC.

### B) General method using 3 equivalents of (III)

It is carried out following the steps described in section A) but using 1.12 mmol of N-(methoxycarbonyl)-L-tert-leucine (III), 0.93 mmol of the corresponding carbodiimide, 0.2 g of 1-[4-(piridyn-2-yl)phenyl-4(S)-hydroxy]-5-(S)-2,5-diamino-6-phenyl-2-azahexane trihydrochloride (II) (trihydrochloride of compound (II) with a 12%wt content of isopropanol, 0.37 mmol) of the trihydrochloride of compound (II), and 2.46 mmol of the corresponding amine.

Table 1 summarizes the reaction conditions of different particular embodiments of the invention carried out following the general processes described above.

**Table 1:**

| E.g | Eq. of (III) | Eq. of carbodiimide | Eq. base | Solvent | Reaction time |
|---|---|---|---|---|---|
| 5 | 3.5 | 2.5 DIC | 6.6 NMM | Toluene | 2h |
| 6 | 3.5 | 2.5 DIC | 6.6 NMM | AcOEt | 3h |
| 7 | 3.0 | 2.5 DIC | 6.6 NMM | CH₂Cl₂ | 24h |
| 8 | 2.5 | 2.6 DIC | 6.6 NMM | CH₂Cl₂ | 3h |
| 9 | 3.5 | 2.5 DIC | 6.6 TEA | CH₂Cl₂ | 1 h |
| 10 | 3.5 | 2.5 DIC | 6.6 TEA | Toluene | 3h |
| 11 | 3.5 | 2.5 DIC | 6.6 DIPEA | CH₂Cl₂ | 22h |
| 12 | 3.5 | 2.5 DCC | 6.6 NMM | CH₂Cl₂ | 3h |
| 13 | 2.5 | 2.5 DCC | 6.6 NMM | CH₂Cl₂ | 3h |

Table 2 summarizes the results obtained regarding to the percentage of atazanavir and the impurities formed during the reaction.

**Table 2:**

| E.g | Atazanavir (HPLC %) | Mono impurity (%) |
|---|---|---|
| 5 | 93.3 | 0.6 |
| 6 | 90.6 | 0.6 |
| 7 | 86.9 | nd |
| 8 | 74 | 1.5 |
| 9 | 93.3 | nd |
| 10 | 92.2 | nd |
| 11 | 82.7 | 7.5 |
| 12 | 88.5 | 0.2 |
| 13 | 76 | nd |

In the previous table nd means non detected.

The previous examples show that high percentages of atazanavir are obtained using DIC in the absence of HOBt and with the tertiary organic amines assayed. Specially good results are obtained with N-methylmorpholine and triethylamine. They also show that a high percentage of atazanavir is also obtained with DCC. The equivalent amount of compound (III) regarding to compound (II) has some influence in the yield obtained, the best results are obtained with 3.5 equivalents of compound (III) per equivalent of compound (II). There are not significant differences when the amount of carbodiimide differs between 2.5 and 3.5 equivalents. Likewise, the process can be carried out with different solvents obtaining similar results.

### Comparative Examples 14-17

Comparative Examples using N-(3-dimethylaminopropyl)-N'-ethylcarbodiimide hydrochloride (WSC) as carbodiimide, or piperidine as base have been included in order to show the effect of the specific selection of the carbodiimide (DIC or DCC) and of the tertiary organic amine. The results are summarized in Table 3.

**Table 3:**

| Eg | Eq. of (III) | Eq. of Carbodiimide | Eq. base | Solvent | Reaction control | | Reaction time |
|---|---|---|---|---|---|---|---|
| | | | | | Atazanavir (HPLC %) | Mono Impurity (%) | |
| 14 | 3.5 | 2.5 DIC | 6.6 piperidine | CH₂Cl₂ | 0 | 0 | 3h |
| 15 | 3.5 | 2.5 WSC | 6.6 NMM | CH₂Cl₂ | 40 | 41 | 24h |
| 16 | 2.5 | 2.6 WSC | 6.6 NMM | CH₂Cl₂ | 11 | 39 | 3h |
| 17 | 2.5 | 2.6 WSC | 6.6 DIPEA | CH₂Cl₂ | 0 | 27 | 3h |

Example 14 was carried out with DIC but using piperidine which is a secondary organic base and no conversion was produced.

Examples 15-17 show the effect of using another carbodiimide. There is either a low percentage of atazanavir or no atazanavir formation at all.

### Comparative Example 18: Preparation of atazanavir using WSC in the presence of HOBt

In comparative Example 18, the condensation between 1-[4-(piridyn-2-yl)phenyl-4(S)-hidroxy]-5-(S)-2,5-diamino-6-phenyl-2-azahexane trihydrochloride (trihydrochloride of compound (II)) and
N-(methoxycarbonyl)-L-tert-leucine (III) was carried out with WSC in the presence of HOBt following the reaction conditions of the process described by Zhongmin Xu et al. in Organic Process Research & Development 2002, vol. 6, pp. 323-328 to show the effect of the presence of HOBt.

A suspension of 1.30 g (2.76 mmol) of 1-[4-(piridyn-2-yl)phenyl-4(S)-hydroxy]-5-(S)-2,5-diamino-6-phenyl-2-azahexane trihydrochloride (trihydrochloride of compound (II)) and 2.93 mL (16.81 mmol) of DIPEA in 5.2 mL of CH₂Cl₂ was transferred over a mixture of 1.30 g (6.86 mmol) of N-(methoxycarbonyl)-L-tert-leucine (III), 1.17 g (7.62 mmol) of HOBt and 1.28 mL (7.22 mmol) of WSC in 1.64 mL of CH₂Cl₂. It was stirred at room temperature during 4 h. The reaction mixture was stirred at room temperature during 4 h, and 30 mL of CH₂Cl₂ were added. The resulting mixture was washed with 25 mL of water (x1), with 25 mL of 10% NaHCO₃ aqueous solution (x1), and with 25 mL of brine (x1). The organic phase was concentrated under vacuum until the formation of a foam. Then, 40 mL of diisopropyl ether were added and the mixture was heated until reflux. 1.761 g (2.49 mmol) of atazanavir were obtained (Yield 91 %). Purity: 88.8% by HPLC.

### Example 19: Preparation of atazanavir as described in Example 18 but without HOBt

It was reproduced the process of Example 18 but without HOBt.

The comparison between examples 18 and 19 is summarized in Table 4.

**Table 4:**

| E.g. | Eq. of (III) | Eq. of Carbodiimide | Eq. base | Solvent | Reaction control (atazanavir HPLC%) | HOBt | Reaction time |
|---|---|---|---|---|---|---|---|
| 18 | 2.5 | 2.6 WSC | 6.1 DIPEA | CH₂Cl₂ | 91 | 2.8 eq. | 4h |
| 19 | 2.5 | 2.6 WSC | 6.1 DIPEA | CH₂Cl₂ | 3 | No | 24h |

It should be noted that the condensation of compound (II) and (III) using WSC/DIPEA in the presence of HOBt gave rise to atazanavir with a yield of 91 %. However, if the reaction is carried out in the absence of HOBt no conversion is produced.

## Claims

1. A process for the preparation of atazanavir of formula (I), or a pharmaceutically salt thereof, or a solvate thereof, 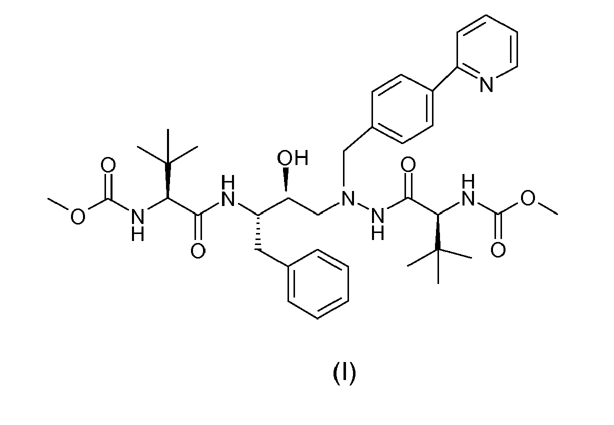 which comprises condensing a compound of formula (II) or a salt thereof, with a compound of formula (III) 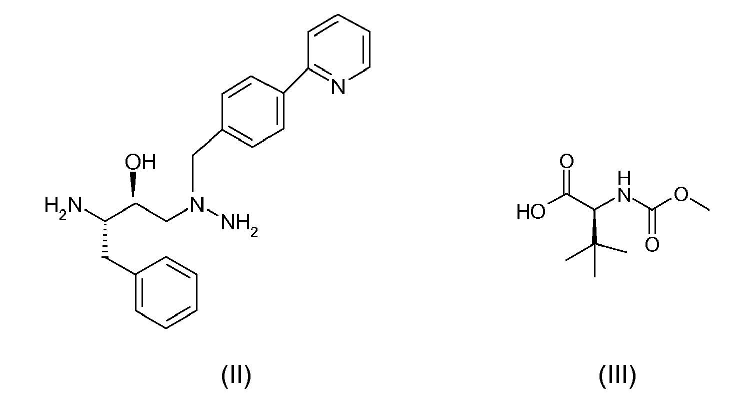 using a carbodiimide selected from N,N-diisopropylcarbodiimide and N,N-dicyclohexylcarbodiimide, and a tertiary organic amine, the condensation being carried out in the absence of 1-hydroxy-benzotriazole.

2. The preparation process according to claim 1, wherein the carbodiimide is N,N-diisopropylcarbodiimide.

3. The preparation process according to any of the claims 1-2, wherein the tertiary organic amine is N-methylmorpholine.

4. The preparation process according to any of the claims 1-2, wherein the tertiary organic amine is triethylamine.

5. The preparation process according to any of the claims 1-2, wherein the tertiary organic amine is N,N-diisopropylethylamine.

6. The preparation process according to any of the claims 1-5, wherein the amount of compound (III) is comprised between 2-20 equivalents of compound (III) per equivalent of compound (II).

7. The preparation process according to claim 6, wherein the reaction is carried out with 3.5 equivalent of compound (III) per equivalent of compound (II).

8. The preparation process according to any of the claims 1-7, wherein the amount of carbodiimide is comprised between 2-20 equivalents of the carbodiimide per equivalent of compound (II).

9. The preparation process according to claim 8, wherein the amount of carbodiimide is comprised between 2.5-3.5 equivalents per equivalent of compound (II).

10. The preparation process according to any of the claims 1-9, wherein the amount of tertiary organic amine is comprised between 2-20 equivalents per equivalent of compound (II).

11. The preparation process according to claim 10, wherein the amount of tertiary organic amine is comprised between 2-8 equivalents per equivalent of compound (II).

12. The preparation process according to claim 10, wherein the amount of tertiary organic amine is comprised between 5-11 equivalents per equivalent of the trihydrochloride of compound (II).

13. The preparation process according to claim 12, wherein the amount of tertiary organic amine is 6.6 equivalents per equivalent of the trihydrochloride of compound (II).

14. The preparation process according to any of the claims 1-13, wherein the solvent is selected from the group consisting of (C₁-C₆)-chlorine containing solvent, (C₆-C₈)-aromatic hydrocarbons, and (C₁-C₆)-alkyl (C₂-C₆)-alkanoates.

15. The preparation process according to claim 14, wherein the solvent is selected from the group consisting of dichloromethane, toluene, and ethyl acetate.
